# EUROPÄISCHE PATENTSCHRIFT

(11) **EP 3 314 654 B1**
(45) Veröffentlichungstag und Bekanntmachung des Hinweises auf die Patenterteilung: **24.07.2019**
(21) Anmeldenummer: 16726879.6
(22) Anmeldetag: 03.06.2016
(51) Int. Cl.: H01L 27/146

(54) **RÖNTGENDETEKTOR MIT KAPAZITÄTSOPTIMIERTEM, LICHTDICHTEM PADAUFBAU**
X-RAY DETECTOR HAVING A CAPACITANCE-OPTIMISED LIGHT-TIGHT PAD STRUCTURE
DÉTECTEUR À RAYONS X AVEC STRUCTURE DE PAVÉ OPAQUE À LA LUMIÈRE ET À CAPACITÉ OPTIMISÉE

(30) Priorität: 28.08.2015 DE 102015216527
(43) Veröffentlichungstag der Anmeldung: 02.05.2018
(73) Patentinhaber: Siemens Healthcare GmbH, 91052 Erlangen (DE)
(72) Erfinder: GROEPL, Martin, 87527 Sonthofen (DE); GÖDERER, Edgar, 91301 Forchheim (DE); SUTTORP, Thomas, 80686 München (DE)
(86) Internationale Anmeldenummer: PCT/EP2016/062650
(87) Internationale Veröffentlichungsnummer: WO 2017/036619

(56) Entgegenhaltungen:
- JP-A- 2015 060 909
- US-A1- 2004 195 640
- US-A1- 2015 054 110
- US-A1- 2015 179 691

## Beschreibung

Die Erfindung betrifft einen Röntgendetektor und ein medizinisches Gerät.

In der Röntgenbildgebung, beispielsweise in der Computertomographie, der Angiographie oder der Radiographie, können zählende direkt-konvertierende Röntgendetektoren verwendet werden. Die Röntgenstrahlung oder die Photonen können durch einen geeigneten Sensor in elektrische Pulse umgewandelt werden. Als Sensormaterial können beispielsweise CdTe, CZT, CdZnTeSe, CdTeSe, CdMnTe, InP, TlBr2, HgI2, GaAs oder andere verwendet werden. Die elektrischen Pulse werden von einer Auswerteelektronik, beispielsweise einem integrierten Schaltkreis (Application Specific Integrated Circuit, ASIC) in einem Substrat, bewertet.

In zählenden Röntgendetektoren mit direkt-konvertierenden Halbleitersensoren werden Elektronen-Loch-Paare durch Absorption von Röntgenquanten im Sensor generiert. Die Elektronen-Loch-Paare werden durch ein an dem Sensor angelegtes elektrisches Feld getrennt. Die Ladungen induzieren in den Elektroden auf beiden Seiten des Sensors einen Ladungspuls. Der Ladungspuls wird in den Detektorelementen des Röntgendetektors von einem Auslesekontakt zu einem Eingang der Signalverarbeitungskette des Detektorelements geleitet. Ein erstes Glied dieser Kette ist typischerweise ein Vorverstärker, beispielsweise ein ladungssensitiver Vorverstärker oder ein Transimpedanzverstärker. Die Eingangskapazität des Vorverstärkers hat Einfluss auf sein Rauschverhalten. Die Eingangskapazität des Vorverstärkers hat ferner Einfluss auf die Auslegung des Vorverstärkers hinsichtlich Leistungsaufnahme und Übertragungsfunktion.

Typischerweise werden Standard-Auslesekontakte verwendet, um den Sensor mit Hilfe von Lotverbindungen, beispielsweise Lötbällen, mit den Eingängen des integrierten Schaltkreises (ASIC) zu verbinden. Dabei werden aus Stabilitätsgründen häufig mehrere oder alle Metalllagen unterhalb des Auslesekontakts mit Via-Verbindungen verbunden. Eine Via-Verbindung ist eine Durchkontaktierung. Die Via-Verbindung stellt eine elektrisch leitende Verbindung dar. Die Via-Verbindung kann zwischen zwei Metalllagen oder Metallisierungsebenen eine elektrisch leitende Verbindung bereitstellen. Der Durchmesser des Auslesekontakts wird von der Größe der zu verwendenden Lötbälle als Lotverbindung zum Sensor bestimmt. Die Lötbälle werden möglichst groß gewählt, um den Abstand zwischen Sensor und oberster Metalllage zu maximieren und um damit die Kapazität zwischen dem Auslesekontakt und dem integrierten Schaltkreis zu minimieren. Um die Kapazität der Via-Verbindungen unterhalb des Auslesekontakts gegenüber benachbarten Leiterbahnen im integrierten Schaltkreis zu verringern, kann um die Via-Verbindung eine metallisierungsfreie Zone eingerichtet werden. Beide Maßnahmen führen zu einem hohen Platzbedarf des Auslesekontakts und den darunterliegenden Strukturen wie beispielsweise Via-Verbindung und metallisierungsfreie Zone. Bei hochintegrierter Pixelelektronik mit kleinen Abständen zwischen den einzelnen Detektorelementen stellen diese Maßnahmen ein Problem dar. Ferner kann durch die metallisierungsfreie Zone Licht, welches den Sensor durchdrungen hat, in den integrierten Schaltkreis gelangen und die Analogelektronik, beispielsweise den Vorverstärker, beeinflussen. Beispielsweise kann das Licht Ladungsträger in den empfindlichen Analogschaltungen generieren. Die Antwort der empfindlichen analogen Verstärkerschaltungen kann dadurch verändert und verschlechtert werden. Daher ist ein lichtdichter Eingang in den Vorverstärker wünschenswert. Der lichtdichte Eingang ist insbesondere für Röntgendetektoren wichtig, bei denen Licht, beispielsweise UV-Licht oder sichtbares Licht, auf die Oberfläche des Sensors gelangen kann. Die Anforderung der Lichtdichtigkeit erschwert die gleichzeitige Erreichung einer minimalen Eingangskapazität.

Die Druckschrift JP 2015 060909 A offenbart eine Halbleitervorrichtung mit einem dreidimensionalen Aufbau, welche ein erstes Substrat mit einem ersten Schaltkreis und ein zweites Substrat mit einem zweiten Schaltkreis umfasst, wobei das erste und das zweite Substrat über ein Verbindungsbereich miteinander leitend verbunden sind, so dass elektrische Signale zwischen dem ersten und dem zweiten Schaltkreis übermittelt werden können. Außerdem weist die Halbleitervorrichtung eine Abschirmungslage, welche benachbarte Verbindungen voneinander elektrisch abgeschirmt.

Die Druckschrift US 2004/195640 A1 offenbart einen Aufbau eines dynamischen Röntgendetektors mit einem großflächigen Sensor, wobei die Pixel über einen Kontaktpunkt und über eine oder mehrere Verbindungslagen mit einem integrierten Schaltkreis verbunden sind.

Es ist Aufgabe der Erfindung, einen Röntgendetektor und ein medizinisches Gerät anzugeben, welche einen lichtdichten Eingang des Vorverstärkers und eine minimale Eingangskapazität des Vorverstärkers ermöglichen.

Die Aufgabe wird erfindungsgemäß gelöst durch einem Röntgendetektor nach Anspruch 1 und ein medizinisches Gerät nach Anspruch 19.

Die Erfindung betrifft einen Röntgendetektor aufweisend ein Substrat mit einer elektrisch leitenden Verbindung zwischen einem Auslesekontakt an bzw. im Bereich der Oberseite des Substrats und einem Eingang eines Vorverstärkers in einer aktiven Lage eines integrierten Schaltkreises. Eine erste elektrisch leitende Verbindung ist zwischen dem Auslesekontakt und einer zweiten elektrisch leitenden Verbindung bereitgestellt. Eine Fläche eines ersten Lichtschutzes ist an der Oberseite des Substrats größer als eine Fläche eines von einem zweiten Lichtschutz seitlich begrenzten lichtdurchlässigen Bereichs im Substrat, sodass die Fläche des ersten Lichtschutzes die Fläche des lichtdurchlässigen Bereichs in einer ersten Projektion entlang der Flächennormale überdeckt. Innerhalb einer zweiten Projektion der Fläche des lichtdurchlässigen Bereichs entlang der Flächennormale und unterhalb des zweiten Lichtschutzes ist die zweite elektrisch leitende Verbindung bereitgestellt. Eine dritte elektrisch leitende Verbindung ist zwischen der zweiten elektrisch leitenden Verbindung und dem Vorverstärker unterhalb des zweiten Lichtschutzes bereitgestellt. Der Eingang des Vorverstärkers ist vor direktem Lichteinfall geschützt.

Das Substrat kann das gesamte Volumen eines ASICs mit darauf aufgebauten Schichten umfassen einschließlich einer ersten Isolationsschicht, den Metalllagen und ein Wafersubstrat mit den aktiven Lagen. Das Wafersubstrat kann beispielsweise auf Silizium basieren. Die Metalllagen können beispielsweise Kupfer oder Aluminium aufweisen. Die Metalllagen oder Metallisierungsebenen können durch ein Dielektrikum, beispielsweise Siliziumoxid, voneinander getrennt sein. Die erste Isolationsschicht kann beispielsweise Siliziumoxid aufweisen.

Der erste Lichtschutz kann der Auslesekontakt sein. Der erste Lichtschutz und der Auslesekontakt können eine Einheit bilden. In einer alternativen Ausführungsform bilden der erste Lichtschutz und der Auslesekontakt keine Einheit, sodass zusätzlich eine elektrisch leitende Verbindung zwischen dem ersten Lichtschutz und dem Auslesekontakt bereitgestellt sein kann. Der erste Lichtschutz kann eine vom Auslesekontakt verschiedene Komponente des Aufbaus sein, beispielsweise die Lotverbindung. Der erste Lichtschutz kann ein Metall aufweisen. Der erste Lichtschutz kann sich an bzw. im Bereich einer dem Sensor zugewandten Oberfläche des Substrats befinden, beispielsweise als Einheit mit dem Auslesekontakt. Der erste Lichtschutz kann sich oberhalb des Auslesekontakts an der Oberfläche des Substrats befinden. Der erste Lichtschutz überdeckt in der ersten Projektion den lichtdurchlässigen Bereich, wobei der lichtdurchlässige Bereich seitlich vom zweiten Lichtschutz begrenzt wird. Die Öffnung des zweiten Lichtschutzes zur Begrenzung des lichtdurchlässigen Bereichs ist kleiner als die Fläche des ersten Lichtschutzes. Die Fläche kann den Flächeninhalt bezeichnen. Die erste elektrisch leitende Verbindung kann aus mehreren Teilen bestehen. Die erste elektrisch leitende Verbindung kann einen Zwischenkontakt aufweisen. Die zweite elektrisch leitende Verbindung kann innerhalb des lichtdurchlässigen Bereichs mit der ersten elektrisch leitenden Verbindung elektrisch leitend verbunden sein. Der zweite Lichtschutz kann eine Metalllage sein. Der lichtdurchlässige Bereich ist frei von Metalllagen und gewährleistet einen Abstand der Metalllagen zur ersten elektrisch leitenden Verbindung. Vorteilhaft wird die Kapazität minimiert. Vorteilhaft wird die Einkopplung von anderen Signalen in den Eingang des Vorverstärkers reduziert oder vermieden. Der lichtdurchlässige Bereich befindet sich vorteilhaft im vom ersten Lichtschutz beschatteten Bereich. Unterhalb des zweiten Lichtschutzes, beispielsweise innerhalb der CMOS-Lagen oder Metalllagen, wird die zweite elektrisch leitende Verbindung mit dem Eingang des Vorverstärkers verbunden. Vorteilhaft wird die Eingangskapazität des Vorverstärkers minimiert. Die elektrisch leitende Verbindung des Auslesekontakts mit dem Eingang des Vorverstärkers wird als Padaufbau bezeichnet, wobei der Padaufbau den Auslesekontakt einschließt. Der Auslesekontakt kann als Ballpad bezeichnet sein. Vorteilhaft wird ein Röntgendetektor mit kapazitätsoptimiertem, lichtdichtem Padaufbau oder mit einer Verbindung des Auslesekontakts mit dem Eingang des Vorverstärkers erreicht. Der Padaufbau ist vorteilhaft undurchlässig für direkten Lichteinfall. Der Padaufbau ist vorteilhaft undurchlässig für UV-Licht oder sichtbares Licht. Die erste elektrisch leitende Verbindung, die zweite elektrisch leitende Verbindung und die dritte elektrisch leitende Verbindung können sich in einem gemeinsamen Schatten des ersten Lichtschutzes, des zweiten Lichtschutzes und des dritten Lichtschutzes befinden, sodass der Eingang des Vorverstärkers besonders vorteilhaft vor Lichteinfall geschützt ist.

Die Erfindung betrifft ferner ein medizinisches Gerät aufweisend einen erfindungsgemäßen Röntgendetektor.

Das medizinische Gerät kann ein Radiographiegerät, ein C-Bogen-Angiographiesystem oder ein Computertomograph sein. In einer bevorzugten Ausführungsform ist das medizinische Gerät ein Computertomograph. Die Vorteile des Röntgendetektors können auf das medizinische Gerät übertragen werden. Vorteilhaft können unabhängig von variablem Lichteinfall auf den Sensor reproduzierbare Ergebnisse oder Röntgenaufnahmen erzielt werden. Der Röntgendetektor kann kleine Detektorelemente aufweisen, vorteilhaft kann eine hohe räumliche Auflösung erreicht werden.

Gemäß einem Aspekt des erfindungsgemäßen Röntgendetektors ist der Auslesekontakt der erste Lichtschutz. Die Größe des Auslesekontakts kann derart gewählt werden, dass sie vorteilhaft der angestrebten Größe der Lötbälle oder Lotverbindungen genügt und gleichzeitig die aktiven Lagen oder den Eingang des Vorverstärkers vorteilhaft vor direktem Lichteinfall schützt.

Gemäß einem Aspekt des erfindungsgemäßen Röntgendetektors ist die erste elektrisch leitende Verbindung und/oder die dritte elektrisch leitende Verbindung eine Via-Verbindung.

Die erste elektrisch leitende Verbindung kann mehrteilig sein. Die einzelnen Teile können eine Via-Verbindung aufweisen. Die Via-Verbindungen können als massive oder hohle, rohrartige Via-Verbindungen ausgestaltet sein. Die Via-Verbindungen können mehrere Metalllagen in einer Stapelanordnung verbinden. Vorteilhaft kann eine hohe Stabilität für die erste elektrisch leitende Verbindung oder die zweite elektrisch leitende Verbindung erreicht werden.

Gemäß einem Aspekt des erfindungsgemäßen Röntgendetektors ist die erste elektrisch leitende Verbindung oder die dritte elektrisch leitende Verbindung zumindest teilweise als mehrlagige Via-Verbindung ausgebildet. Die mehrlagigen Via-Verbindungen nutzen Teilbereiche der Metalllagen, welche von den Metalllagen, beispielsweise dem zweiten Lichtschutz, horizontal durch einen Abstand getrennt sind. Vorteilhaft können Verbindungen senkrecht zu den Metalllagen während des Aufbringens der Metalllagen realisiert werden. Vorteilhaft kann die Grundfläche der Via-Verbindung zwischen dem Zwischenkontakt und der zweiten elektrisch leitenden Verbindung kleiner als die Fläche des Zwischenkontakts sein.

Gemäß einem Aspekt des erfindungsgemäßen Röntgendetektors ist zwischen der Oberseite des Substrats und dem zweiten Lichtschutz eine erste Isolationsschicht bereitgestellt.

Es kann eine dicke erste Isolationsschicht zwischen der Oberseite des Substrats und dem zweiten Lichtschutz bereitgestellt sein. Die Isolationsschicht kann beispielsweise zwischen 10µm und 200µm dick sein. Es kann eine dicke erste Isolationsschicht zwischen dem ersten Lichtschutz und dem zweiten Lichtschutz bereitgestellt sein. Die erste Isolationsschicht kann sich auf der gesamten horizontalen Breite parallel zum zweiten Lichtschutz oder der Oberfläche des Substrats erstrecken. Die erste Isolationsschicht kann vorteilhaft in billigen oder günstigen Prozessschritten aufgebracht werden. Vorteilhaft kann die erste Isolationsschicht die mechanische Stabilität des Aufbaus erhöhen. Vorteilhaft kann die Kapazität des Auslesekontakts minimiert werden.

Gemäß einem Aspekt des erfindungsgemäßen Röntgendetektors weist die erste elektrisch leitende Verbindung einen Zwischenkontakt auf. Der zweite Lichtschutz weist mehrere Metalllagen auf. Der Zwischenkontakt ist in einer oberen Metalllage bereitgestellt.

Der Zwischenkontakt kann sich in einer oberen Metalllage befinden. Bevorzugt befindet sich der Zwischenkontakt in der obersten Metalllage, welche dem Auslesekontakt am nächsten ist und vorteilhaft am weitesten von den aktiven Lagen entfernt ist. Der Zwischenkontakt ist auf eine Fläche innerhalb der Metalllage begrenzt und von einer Aussparung umgeben. Der Abstand zwischen einer oberen Metalllage und den aktiven Lagen kann gering sein. Vorteilhaft kann Größe und Form des Zwischenkontakts unabhängig vom Auslesekontakt gewählt werden.

Gemäß einem Aspekt des erfindungsgemäßen Röntgendetektors weist der Zwischenkontakt in einer Projektion entlang der Flächennormale eine kleinere Fläche als der Auslesekontakt auf.

Die Fläche des Zwischenkontakts ist klein und entspricht bevorzugt einer minimalen technologisch erlaubten Größe. Die Fläche des Zwischenkontakts ist kleiner als der Auslesekontakt. Die Form des Zwischenkontakts kann rund oder eckig sein. In einer bevorzugten Ausführungsform ist der Auslesekontakt kleiner als 50 Prozent der Fläche des Auslesekontakts. Vorteilhaft kann die Größe des Zwischenkontakts kleiner als die Größe des Auslesekontakts gewählt werden. Der Abstand zwischen einer oberen Metalllage und den aktiven Lagen kann gering sein. Vorteilhaft kann die Kapazität, insbesondere die parasitäre Kapazität, durch die im Vergleich zum Auslesekontakt kleine Fläche des Zwischenkontakts reduziert werden. Durch die geringe Größe des Zwischenkontakts kann pro Detektorelement mehr Platz für Schaltungen vorteilhaft zur Verfügung stehen. Falls das Platzieren von Schaltungen unterhalb eines Kontakts, beispielsweise des Zwischenkontakts, verboten ist, ist die geringere Größe des Zwischenkontakts besonders vorteilhaft, weil der verbotene Bereich verkleinert wird. Vorteilhaft kann die Größe des Zwischenkontakts deutlich kleiner als die Größe des Auslesekontakts oder die Größe des Lötballs oder der Lotverbindung gewählt werden. Die Größe des Zwischenkontakts kann beispielsweise im Bereich von 10µm bis 50µm liegen.

Gemäß einem Aspekt des erfindungsgemäßen Röntgendetektors verbindet die erste elektrisch leitende Verbindung vorteilhaft den Auslesekontakt und die zweite elektrisch leitende Verbindung elektrisch leitend.

Gemäß einem Aspekt des erfindungsgemäßen Röntgendetektors weist die erste elektrisch leitende Verbindung eine Via-Verbindung zwischen dem Auslesekontakt und dem Zwischenkontakt, den Zwischenkontakt und eine mehrlagige Via-Verbindung zwischen dem Zwischenkontakt und der zweiten elektrisch leitenden Verbindung auf. Anders ausgedrückt können eine Via-Verbindung zwischen dem Auslesekontakt und dem Zwischenkontakt, der Zwischenkontakt selber, sowie eine mehrlagige Via-Verbindung zwischen dem Zwischenkontakt und der zweiten elektrisch leitenden Verbindung gemeinsam die erste elektrisch leitende Verbindung oder Teile davon bilden.

Die erste elektrisch leitende Verbindung kann den Zwischenkontakt aufweisen. Der Auslesekontakt und der Zwischenkontakt können über eine Via-Verbindung verbunden sein. Diese Via-Verbindung kann Teil der ersten elektrisch leitenden Verbindung sein. Der Zwischenkontakt kann ferner über eine mehrlagige Via-Verbindung mit der zweiten elektrisch leitenden Verbindung verbunden sein, wobei sich die zweite elektrisch leitende Verbindung in einer unteren Metalllage befindet. Die mehrlagige Via-Verbindung kann eine runde oder eckige Grundfläche aufweisen. In einer bevorzugten Ausführungsform kann die Grundfläche ein Vieleck, beispielsweise ein Achteck, sein. Die Grundfläche kann vorteilhaft kleiner als die Fläche des Zwischenkontakts gewählt werden, sodass trotzdem ausreichend mechanische Stabilität gewährleistet ist.

Gemäß einem Aspekt des erfindungsgemäßen Röntgendetektors ist eine aktive Lage des integrierten Schaltkreises durch den ersten Lichtschutz, den zweiten Lichtschutz oder einen dritten Lichtschutz vor direktem Lichteinfall von der Oberseite geschützt.

Vorteilhaft wird das generieren von Ladungsträgern in den empfindlichen Analogschaltungen oder in der Umgebung der aktiven Lagen reduziert oder vermieden. Die Antwort der empfindlichen analogen Verstärkerschaltungen kann dadurch vorteilhaft stabilisiert werden.

Der dritte Lichtschutz kann eine Durchführung der dritten elektrisch leitendenden Verbindung bereitstellen. Der dritte Lichtschutz begrenzt den lichtdurchlässigen Bereich oberhalb der aktiven Lage, sodass die aktive Lage vorteilhaft vor Lichteinfall geschützt ist.

Gemäß einem Aspekt des erfindungsgemäßen Röntgendetektors ist der zweite Lichtschutz oder der dritte Lichtschutz eine Metalllage. Vorteilhaft kann eine Metalllage durch geeignete Positionierung und Beabstandung zu der aktiven Lage als Lichtschutz genutzt werden. Vorteilhaft werden keine zusätzlichen Prozessschritte während der Herstellung benötigt.

Gemäß einem Aspekt des erfindungsgemäßen Röntgendetektors weist der dritte Lichtschutz eine Aussparung auf. Vorteilhaft kann die Aussparung die Eingangskapazität des Vorverstärkers minimieren. Die Aussparung liegt dabei innerhalb eines abgeschatteten Bereichs, beispielsweise in einer Projektion der Fläche des Auslesekontakts oder des Zwischenkontakts entlang der Flächennormalen. Die Fläche der Aussparung kann kleiner als die Fläche des Auslesekontakts oder des Zwischenkontakts sein, sodass die aktiven Lagen vorteilhaft vor Lichteinfall geschützt sind.

Gemäß einem Aspekt des erfindungsgemäßen Röntgendetektors ist der dritte Lichtschutz eine Metalllage unterhalb des zweiten Lichtschutzes. Diese Metalllage kann so ausgestaltet sein, dass der lichtdurchlässige Bereich hin zu der aktiven Lage begrenzt ist. Vorteilhaft wird Lichteinfall in die aktive Lage vermieden. Diese Metalllage ist undurchlässig für Licht.

Gemäß einem Aspekt des erfindungsgemäßen Röntgendetektors ist die zweite elektrisch leitende Verbindung als Metalllage ausgebildet. Vorteilhaft kann die zweite elektrisch leitende Verbindung innerhalb eines Prozessschrittes zur Herstellung einer Metalllage hergestellt werden. Die zweite elektrisch leitende Verbindung ist von anderen Bereichen der Metalllage durch isolierendes Material getrennt und beabstandet.

Gemäß einem Aspekt des erfindungsgemäßen Röntgendetektors ist ein vierter Lichtschutz an der Oberseite des Substrates bereitgestellt. Der vierte Lichtschutz kann eine Umverdrahtungsschicht sein. Der vierte Lichtschutz kann metallisch und vorteilhaft undurchlässig für Licht sein. Der vierte Lichtschutz kann vorteilhaft vor Lichteinfall schützen. Vorteilhaft kann Lichteinfall, der nicht senkrecht auf die Oberfläche einfällt, gehindert werden, in den lichtdurchlässigen Bereich zu gelangen. Vorteilhaft wird die parasitäre Kapazität des vierten Lichtschutzes durch die erste Isolationsschicht reduziert.

Gemäß einem Aspekt des erfindungsgemäßen Röntgendetektors ist eine zweite Isolationsschicht an der Oberseite des Substrates außerhalb der Fläche des Auslesekontakts bereitgestellt und der vierte Lichtschutz zumindest teilweise von der zweiten Isolationsschicht bedeckt. Vorteilhaft wird der vierte Lichtschutz gegenüber Einflüssen von außerhalb des Substrats geschützt. Die zweite Isolationsschicht und der vierte Lichtschutz tragen vorteilhaft zur mechanischen Stabilität bei.

Gemäß einem Aspekt des erfindungsgemäßen Röntgendetektors folgen der erste Lichtschutz, die erste Isolationsschicht, der zweite Lichtschutz und der Vorverstärker in einer Stapelanordnung aufeinander. Die Oberseite des Substrats ist dem Sensor zugewandt, damit befindet sich der erste Lichtschutz oben. Der Vorverstärker befindet sich relativ zum ersten Lichtschutz unten oder unterhalb des ersten Lichtschutzes, und damit in der abgewandten Richtung relativ zum Sensor. Die einzelnen Komponenten der Stapelanordnung können unterschiedliche horizontale und vertikale Ausdehnungen aufweisen. Der erste Lichtschutz und der zweite Lichtschutz sind derart relativ zum lichtdurchlässigen Bereich und dem Eingang des Vorverstärkers angeordnet, dass der Eingang des Vorverstärkers vor direktem Lichteinfall geschützt ist.

Gemäß einem Aspekt des erfindungsgemäßen Röntgendetektors ist zwischen der ersten Isolationsschicht und den aktiven Lagen eine obere Metalllage, eine mittlere Metalllage und eine untere Metalllage in einer Stapelanordnung angeordnet.

Die obere Metalllage befindet sich in einem geringeren Abstand zur Oberseite des Substrats oder zum Sensor als die mittlere Metalllage oder die untere Metalllage. Die obere Metalllage, die mittlere Metalllage und die untere Metalllage sind undurchlässig für Licht. Vorteilhaft können die erste elektrisch leitende Verbindung, die zweite elektrisch leitende Verbindung und die dritte elektrisch leitende Verbindung zumindest teilweise während der Herstellungsschritte zum Herstellen der Metalllagen hergestellt werden.

Gemäß einem Aspekt des erfindungsgemäßen medizinischen Geräts ist das medizinische Gerät ein Computertomograph. Vorteilhaft kann die Antwort der empfindlichen analogen Verstärkerschaltungen für alle Projektionen oder alle Aufnahmen stabilisiert werden. Vorteilhaft sind die rekonstruierten Schichtbilder, dreidimensionalen oder vierdimensionalen Darstellung in einer Bildqualität unabhängig vom Lichteinfall bereitstellbar.

Nachfolgend werden Ausführungsbeispiele der Erfindung anhand von Zeichnungen näher erläutert. Hierbei zeigt:
FIG 1 schematisch ein Konzept eines erfindungsgemäßen Röntgendetektors gemäß einer ersten Ausführungsform;
FIG 2 schematisch ein Konzept eines erfindungsgemäßen Röntgendetektors gemäß einer zweiten Ausführungsform;
FIG 3 schematisch ein Konzept eines erfindungsgemäßen Röntgendetektors gemäß einer dritten Ausführungsform;
FIG 4 schematisch ein Detektormodul mit einer Anordnung von erfindungsgemäßen Röntgendetektoren; und
FIG 5 schematisch eine Darstellung eines erfindungsgemäßen Computertomographen.

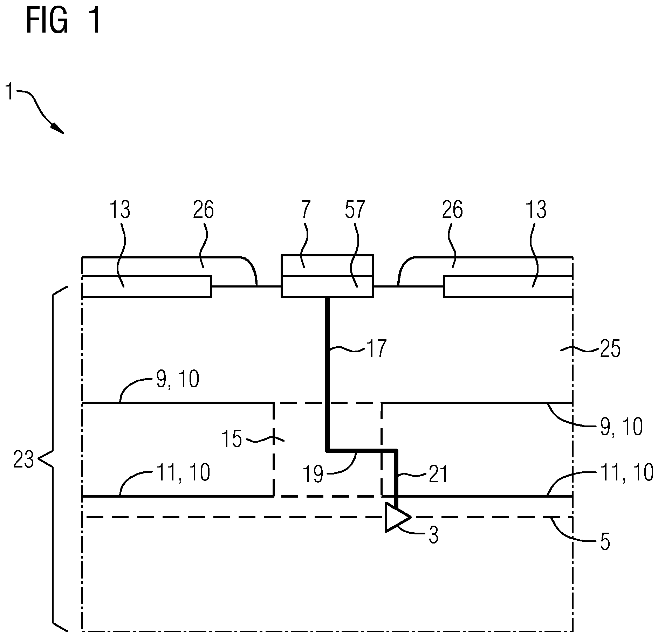

Die Fig. 1 zeigt eine beispielhafte Ausführung eines erfindungsgemäßen Röntgendetektors 1 gemäß einer ersten Ausführungsform. Das Substrat 23 umfasst das gesamte Volumen eines ASICs mit darauf aufgebauten Schichten umfassen einschließlich einer ersten Isolationsschicht 25, den Metalllagen 10 und ein Wafersubstrat mit der aktiven Lage 5. Das Wafersubstrat basiert beispielsweise auf Silizium. Die Metalllagen 10 weisen beispielsweise Kupfer oder Aluminium auf. Die Metalllagen 10 oder Metallisierungsebenen sind durch ein Dielektrikum, beispielsweise Siliziumoxid, voneinander getrennt. Die erste Isolationsschicht 25 kann beispielsweise Siliziumoxid aufweisen. Der erste Lichtschutz 7 befindet sich oberhalb des Auslesekontakts 57. Der Auslesekontakt 57 ist an der Oberfläche des Substrats 23. Der erste Lichtschutz 7 und der Auslesekontakt 57 können eine Einheit bilden. Der erste Lichtschutz 7 ist über den Auslesekontakt 57, eine erste elektrisch leitende Verbindung 17, eine zweite elektrisch leitende Verbindung 19 und eine dritte elektrisch leitende Verbindung 21 mit dem Vorverstärker 3 elektrisch leitend verbunden. Der erste Lichtschutz 7 weist ein Metall auf. Der Vorverstärker 3 befindet sich in der aktiven Lage 5. Der Röntgendetektor 1 weist ferner den zweiten Lichtschutz 9 auf, der den lichtdurchlässigen Bereich 15 seitlich begrenzt. Die erste elektrisch leitende Verbindung 17 befindet sich zumindest teilweise innerhalb der ersten Isolationsschicht 25 und dem lichtdurchlässigen Bereich 15. Die zweite elektrisch leitende Verbindung 19 befindet sich zumindest teilweise im lichtdurchlässigen Bereich 15 und in einem Bereich unterhalb des zweiten Lichtschutzes 9. Der Bereich unterhalb des zweiten Lichtschutzes ist der Bereich innerhalb einer Projektion des zweiten Lichtschutzes 9 entlang der Flächennormalen. Die dritte elektrisch leitende Verbindung 21 verbindet elektrisch leitend die zweite elektrisch leitende Verbindung 19 mit dem Vorverstärker 3. Die dritte elektrisch leitende Verbindung befindet sich unterhalb des zweiten Lichtschutzes 9. Zwischen dem zweiten Lichtschutz 9, 10 und der aktiven Lage 5 befindet sich der dritte Lichtschutz 10, 11. Er weist eine Durchführung für die dritte elektrisch leitende Verbindung 21 auf.

Ein vierter Lichtschutz 13 befindet sich an der Oberfläche des Substrats 23. Eine zweite Isolationsschicht 26 befindet sich über dem vierten Lichtschutz 13 an der Oberfläche des Substrats 23 sowie auf der Oberfläche des Substrats 23, wobei der erste Lichtschutz 7 ausgespart ist.

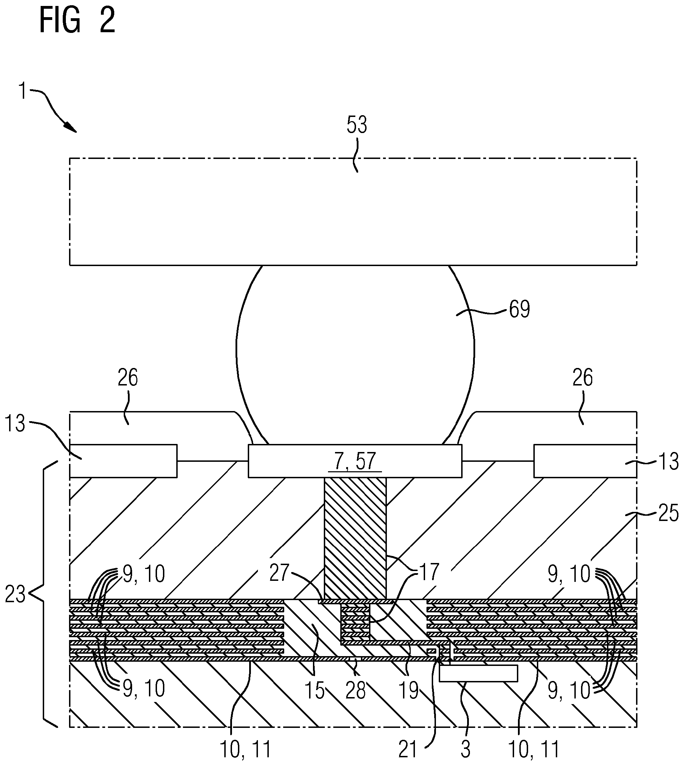

Die Fig. 2 zeigt eine beispielhafte Ausführung eines erfindungsgemäßen Röntgendetektors 1 gemäß einer zweiten Ausführungsform. Der Röntgendetektor 1 weist einen Sensor 53, beispielsweise aufweisend CdTe, CZT, CdZnTeSe, CdTeSe, CdMnTe, InP, TlBr2, HgI2 oder GaAs, und ein Substrat 23 mit einer aktiven Lage 5 auf. Der Sensor 53 ist über eine Lotverbindung 69, beispielsweise einen Lötball oder eine säulenförmige, kupferhaltige Verbindung (copper pillar, nicht gezeigt), mit dem Auslesekontakt 7, 57 elektrisch leitend verbunden. Der Auslesekontakt 57 bildet eine Einheit mit dem ersten Lichtschutz 7. Der Auslesekontakt 7, 57 bilden somit gemeinsam den ersten Lichtschutz 7. An der Oberfläche des Substrats 23 befindet sich der vierte Lichtschutz 13. Die zweite Isolationsschicht 26 bedeckt den vierten Lichtschutz 13 und zumindest teilweise die Oberfläche des Substrats 23. Der vierte Lichtschutz 13 ist eine Umverdrahtungsschicht. Unterhalb des Auslesekontakts 7, 57, dem vierten Lichtschutz 13 und der zweiten Isolationsschicht 26 befindet sich die erste Isolationsschicht 25. Die erste Isolationsschicht ist durch den zweiten Lichtschutz 25 und den lichtdurchlässigen Bereich 15 nach unten begrenzt. Der zweite Lichtschutz 9, 10 weist mehrere Lagen auf. Die oberste Lage des zweiten Lichtschutzes 9,10 ist Teil der obersten Metalllage 10. Der Zwischenkontakt 27 ist ebenfalls Teil der obersten Metalllage 10. Die Fläche des Zwischenkontakts 27 ist kleiner als die Fläche des Auslesekontakts 7, 57. Der Auslesekontakt 7, 57 ist elektrisch leitend über eine Via-Verbindung, welche Teil der ersten elektrisch leitenden Verbindung 17 ist, mit dem Zwischenkontakt 27 verbunden. Der lichtdurchlässige Bereich 15 ist seitlich durch die mehreren Lagen des zweiten Lichtschutzes 9, 10 begrenzt. Innerhalb des lichtdurchlässigen Bereichs 15 ist der Zwischenkontakt 27 über eine mehrlagige Via-Verbindung, welche Teil der ersten elektrisch leitenden Verbindung 17 ist, elektrisch leitend mit der zweiten elektrisch leitenden Verbindung 19 verbunden. Die zweite elektrisch leitende Verbindung 19 verbindet die erste elektrisch leitenden Verbindung 17 mit der dritten elektrisch leitenden Verbindung 21, welche sich unterhalb des zweiten Lichtschutzes 9, 10 befindet. Der Eingang des Vorverstärkers 3 befindet sich unterhalb des dritten Lichtschutzes 10, 11. Der dritte Lichtschutz 10, llbefindet sich unterhalb der mehreren Lagen des zweiten Lichtschutzes 9, 10. Die dritte elektrisch leitende Verbindung 21 verbindet die zweite elektrisch leitende Verbindung 19 mit dem Eingang des Vorverstärkers 3 in der aktiven Lage 5. Unterhalb des Zwischenkontakts 27 und unterhalb der mehrlagigen Via-Verbindung, welche Teil der ersten elektrisch leitenden Verbindung 17 ist, ist der dritten Lichtschutz mit einer Aussparung 28 ausgebildet.

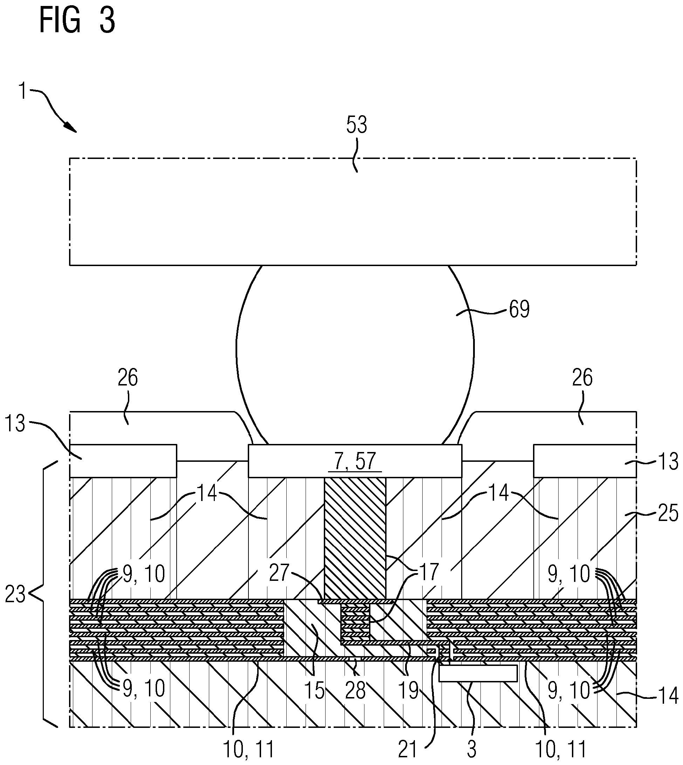

Die Fig. 3 zeigt eine beispielhafte Ausführung eines erfindungsgemäßen Röntgendetektors gemäß einer dritten Ausführungsform. In einer Projektion entlang der Flächennormalen werfen der erste Lichtschutz 7 als Einheit mit dem Auslesekontakt 57, der zweite Lichtschutz 9, 10, der dritte Lichtschutz 10, 11 und der vierte Lichtschutz 13 Schatten 14, sodass eine vollkommene Beschattung der aktiven Lagen 5 erreicht wird.

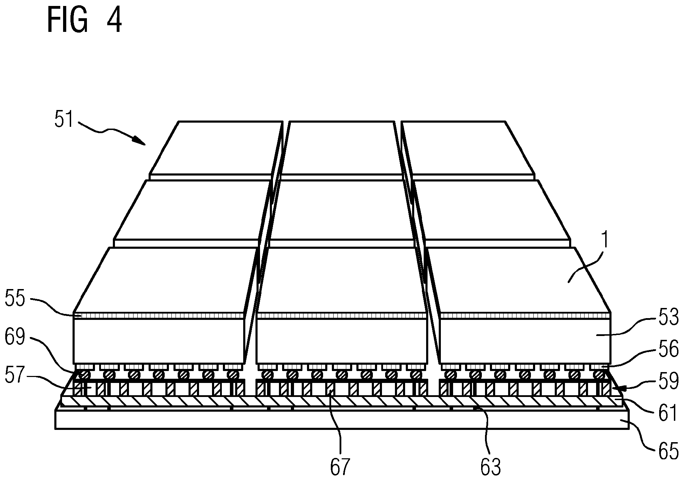

Die Fig. 4 zeigt eine beispielhafte Ausführung eines Detektormoduls 51 mit einer Anordnung von erfindungsgemäßen Röntgendetektoren 1. In einer bevorzugten Ausführungsform weist der Röntgendetektor 1 eine zweidimensionale Matrix oder Anordnung einer Mehrzahl von Pixeln oder Subpixeln auf. Die Anzahl der Subpixel kann beispielsweise im Bereich von 100 bis mehrere Tausend liegen. Der Röntgendetektor 1 weist einen Sensor 53 auf. Der Sensor 53 kann als flächenhafter Direktkonverter, beispielsweise aufweisend CdTe, CZT, CdZnTeSe, CdTeSe, CdMnTe, InP, TlBr2, HgI2, GaAs oder andere als Konvertermaterial, ausgebildet sein. Die Oberseite des Sensors 53 weist eine Top-Elektrode 55 auf. Die Unterseite des Sensors 53 weist eine zweidimensionale Anordnung von Kontakten 56 auf. Die Kontakte 56 sind über Lotverbindungen 69 mit den Auslesekontakten 57 und den Pixelelektroniken 67 im Substrat 59 verbunden. Die Lotverbindungen 69 können beispielsweise als Lötbälle (bump bonds) oder Lotmaterial in Verbindung mit Kupfersäulen (copper pillars) ausgebildet sein. Die Anzahl der Kontakte 56, die Anzahl der Lotverbindungen 69, die Anzahl der Auslesekontakte 57 und die Anzahl der Pixelelektroniken 67 im Substrat 59 sind gleich. Das elektrische Feld zwischen der Top-Elektrode 55 und einem Kontakt 56 bestimmt ein sensitives Detektionsvolumen. Die Einheit aus einem Detektionsvolumen, einem Kontakt 56, einer Lotverbindung 69, einem Auslesekontakt 57 und einer mit dem Auslesekontakt 57 verbundenen Pixelelektronik 67 bildet einen Pixel oder Subpixel. Das Substrat 59 ist an der Unterseite mit einer Trägerplatte 61 verbunden. Das Substrat 59 ist über TSV-Verbindungen 63 durch die Trägerplatte 61 hindurch mit einer peripheren Elektronik 65 verbunden. Das Substrat 59 weist eine elektrisch leitende Verbindung zwischen einem Auslesekontakt 57 an der Oberseite des Substrats 23 und einem Eingang eines Vorverstärkers 3 in einer aktiven Lage 5 eines integrierten Schaltkreises mit dem erfindungsgemäßen kapazitätsoptimierten, lichtdichten Padaufbau auf.

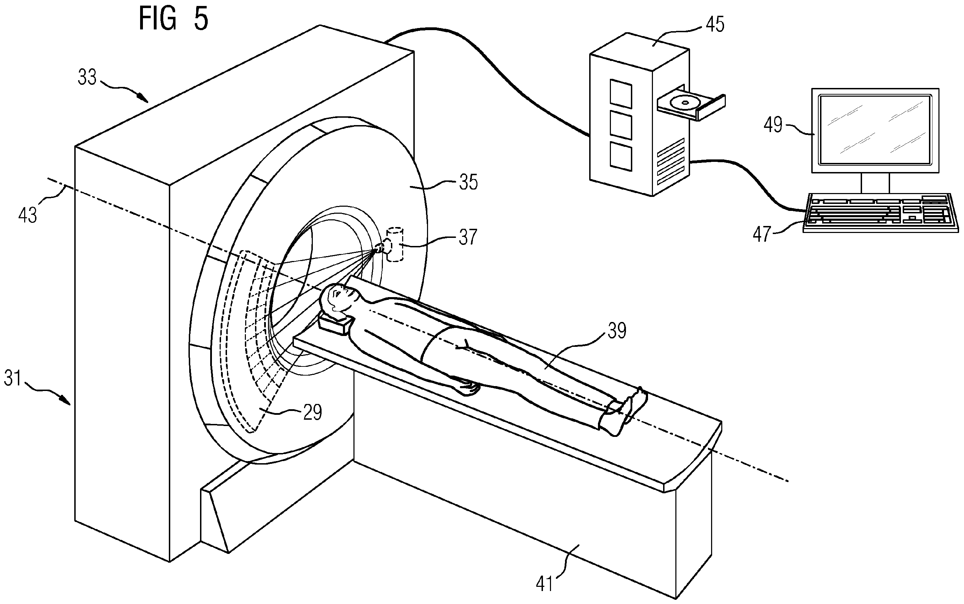

Die Fig. 5 zeigt eine beispielhafte Ausführung eines erfindungsgemäßen Computertomographen 31 mit einer erfindungsgemäßen Detektorvorrichtung 29. Die Detektorvorrichtung 29 weist den erfindungsgemäßen Röntgendetektor 1 auf. Die Detektorvorrichtung 29 kann mehrere Detektormodule 51 aufweisen die mindestens einen Röntgendetektor 1 aufweisen. Bevorzugt weisen die Detektormodule 51 eine Mehrzahl an Röntgendetektoren 1 in einer zweidimensionale Matrix oder Anordnung auf. Der Computertomograph 31 beinhaltet eine Gantry 33 mit einem Rotor 35. Der Rotor 35 umfasst eine Röntgenquelle 37 und die erfindungsgemäße Detektorvorrichtung 29. Der Patient 39 ist auf der Patientenliege 41 gelagert und ist entlang der Rotationsachse z 43 durch die Gantry 33 bewegbar. Zur Steuerung und Berechnung der Schnittbilder wird eine Recheneinheit 45 verwendet. Eine Eingabeeinrichtung 47 und eine Ausgabevorrichtung 49 sind mit der Recheneinheit 45 verbunden.

Obwohl die Erfindung im Detail durch das bevorzugte Ausführungsbeispiel näher illustriert wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgendetektor (1) aufweisend ein Substrat (23) mit einer elektrisch leitenden Verbindung zwischen einem Auslesekontakt (57), welcher über eine Lotverbindung mit einem Sensor (53) elektrisch leitend verbunden ist, im Bereich der Oberseite des Substrats (23) und einem Eingang eines Vorverstärkers (3) in einer aktiven Lage (5) eines integrierten Schaltkreises, wobei
a. eine erste elektrisch leitende Verbindung (17) zwischen dem Auslesekontakt (57) und einer zweiten elektrisch leitenden Verbindung (19) bereitgestellt ist,
b. eine Fläche eines ersten Lichtschutzes (7) an der Oberseite des Substrats (23) größer ist als eine Fläche eines von einem zweiten Lichtschutz (9) seitlich begrenzten lichtdurchlässigen Bereichs (15) im Substrat (23), sodass die Fläche des ersten Lichtschutzes (7) die Fläche des lichtdurchlässigen Bereichs (15) in einer ersten Projektion entlang der Flächennormale überdeckt und sich der lichtdurchlässige Bereich (15) im vom ersten Lichtschutz (7) beschatteten Bereich befindet,
c. sich der erste Lichtschutz (7) oberhalb des Auslesekontakts (57) an der Oberfläche des Substrats (23) befindet oder der erste Lichtschutz (7) eine Einheit mit dem Auslesekontakt (57) bildet,
d. innerhalb einer zweiten Projektion der Fläche des lichtdurchlässigen Bereichs (15) entlang der Flächennormale und unterhalb des zweiten Lichtschutzes (9) die zweite elektrisch leitende Verbindung (19) bereitgestellt ist, und
e. eine dritte elektrisch leitende Verbindung (21) zwischen der zweiten elektrisch leitenden Verbindung (19) und dem Vorverstärker (3) unterhalb des zweiten Lichtschutzes (9) bereitgestellt ist,
sodass der Eingang des Vorverstärkers (3) vor direktem Lichteinfall geschützt ist.

2. Röntgendetektor (1) nach Anspruch 1, wobei der Auslesekontakt (57) der erste Lichtschutz (7) ist.

3. Röntgendetektor (1) nach einem der vorangehenden Ansprüche, wobei die erste elektrisch leitende Verbindung (17) oder die dritte elektrisch leitende Verbindung (21) eine Via-Verbindung ist.

4. Röntgendetektor (1) nach Anspruch 3, wobei die erste elektrisch leitende Verbindung (17) oder die dritte elektrisch leitende Verbindung (21) zumindest teilweise als mehrlagige Via-Verbindung ausgebildet ist.

5. Röntgendetektor (1) nach einem der vorangehenden Ansprüche, wobei zwischen der Oberseite des Substrats (23) und dem zweiten Lichtschutz (9) eine erste Isolationsschicht (25) bereitgestellt ist.

6. Röntgendetektor (1) nach einem der vorangehenden Ansprüche, wobei die erste elektrisch leitende Verbindung (17) einen Zwischenkontakt (27) aufweist, der zweite Lichtschutz (9) mehrere Metalllagen (10) aufweist und der Zwischenkontakt (27) in einer oberen Metalllage (10) bereitgestellt ist.

7. Röntgendetektor (1) nach Anspruch 6, wobei der Zwischenkontakt (27) in einer Projektion entlang der Flächennormale eine kleinere Fläche als der Auslesekontakt (57) aufweist.

8. Röntgendetektor (1) nach einem der vorangehenden Ansprüche, wobei die erste elektrisch leitende Verbindung (17) den Auslesekontakt (57) und die zweite elektrisch leitende Verbindung (19) elektrisch leitend verbindet.

9. Röntgendetektor (1) nach einem der Ansprüche 6 bis 8, wobei die erste elektrisch leitende Verbindung (17) eine Via-Verbindung zwischen dem Auslesekontakt(57) und dem Zwischenkontakt (27), den Zwischenkontakt (27) und eine mehrlagige Via-Verbindung zwischen dem Zwischenkontakt (27) und der zweiten elektrisch leitenden Verbindung (19) aufweist.

10. Röntgendetektor (1) nach einem der vorangehenden Ansprüche, wobei eine aktive Lage (5) des integrierten Schaltkreises durch den ersten Lichtschutz (7), den zweiten Lichtschutz (9) oder einen dritten Lichtschutz (11) vor direktem Lichteinfall von der Oberseite geschützt ist.

11. Röntgendetektor (1) nach Anspruch 10, wobei der zweite Lichtschutz (9, 10) oder der dritte Lichtschutz (10, 11) eine Metalllage (10) ist.

12. Röntgendetektor (1) nach Anspruch 10 oder 11, wobei der dritte Lichtschutz (11) eine Aussparung aufweist.

13. Röntgendetektor (1) nach einem der Ansprüche 10 bis 12, wobei der dritte Lichtschutz (10, 11) eine Metalllage (10) unterhalb des zweiten Lichtschutzes (9, 10) ist.

14. Röntgendetektor (1) nach einem der vorangehenden Ansprüche, wobei die zweite elektrisch leitende Verbindung (19) als Metalllage (10) ausgebildet ist.

15. Röntgendetektor (1) nach einem der vorangehenden Ansprüche, wobei ein vierter Lichtschutz (13) an der Oberseite des Substrates (23) bereitgestellt ist.

16. Röntgendetektor (1) nach einem der vorangehenden Ansprüche, wobei eine zweite Isolationsschicht (26) an der Oberseite des Substrates (23) außerhalb der Fläche des Auslesekontakts (57) bereitgestellt ist und der vierte Lichtschutz (13) zumindest teilweise von der zweiten Isolationsschicht (26) bedeckt ist.

17. Röntgendetektor (1) nach einem der vorangehenden Ansprüche, wobei der erste Lichtschutz (7), die erste Isolationsschicht (25), der zweite Lichtschutz (9) und eine aktive Lage (5) mit dem Vorverstärker (3) in einer Stapelanordnung aufeinanderfolgen.

18. Röntgendetektor (1) nach einem der vorangehenden Ansprüche, wobei zwischen der ersten Isolationsschicht (25) und den aktiven Lagen (5) eine obere Metalllage (10), eine mittlere Metalllage (10) und eine untere Metalllage (10) in einer Stapelanordnung angeordnet ist.

19. Medizinisches Gerät aufweisend einen Röntgendetektor (1) nach einem der vorangehenden Ansprüche.

20. Medizinisches Gerät nach Anspruch 19, wobei das medizinische Gerät ein Computertomograph (31) ist.

## Claims

1. X-ray detector (1) having a substrate (23) which has an electrically conductive connection between a read-out contact (57), which has an electrically conductive connection to a sensor (53) via a soldered connection, in the region of the top side of the substrate (23) and an input of a pre-amplifier (3) in an active layer (5) of an integrated circuit, wherein
a. a first electrically conductive connection (17) is provided between the read-out contact (57) and a second electrically conductive connection (19),
b. a surface of a first light protection (7) at the top side of the substrate (23) is larger than a surface of a light-permeable region (15) which is in the substrate (23) and laterally delimited by a second light protection (9), so that the surface of the first light protection (7) covers the surface of the light-permeable region (15) in a first projection along the surface normal and the light-permeable region (15) is situated in that region which is shaded by the first light protection (7),
c. the first light protection (7) is situated above the read-out contact (57) at the upper surface of the substrate (23) or the first light protection (7) forms a unit with the read-out contact (57),
d. the second electrically conductive connection (19) is provided within a second projection of the surface of the light-permeable region (15) along the surface normal and below the second light protection (9),
e. a third electrically conductive connection (21) between the second electrically conductive connection (19) and the pre-amplifier (3) is provided below the second light protection (9),
so that the input of the pre-amplifier (3) is protected against direct incidence of light.

2. X-ray detector (1) according to claim 1, wherein the read-out contact (57) is the first light protection (7).

3. X-ray detector (1) according to one of the preceding claims, wherein the first electrically conductive connection (17) or the third electrically conductive connection (21) is a via connection.

4. X-ray detector (1) according to claim 3, wherein the first electrically conductive connection (17) or the third electrically conductive connection (21) is designed at least partly as a multilayer via connection.

5. X-ray detector (1) according to one of the preceding claims, wherein a first insulation layer (25) is provided between the top side of the substrate (23) and the second light protection (9).

6. X-ray detector (1) according to one of the preceding claims, wherein the first electrically conductive connection (17) has an intermediate contact (27), the second light protection (9) has a plurality of metal layers (10), and the intermediate contact (27) is provided in an upper metal layer (10).

7. X-ray detector (1) according to claim 6, wherein the intermediate contact (27) has a smaller surface than the read-out contact (57) in a projection along the surface normal.

8. X-ray detector (1) according to one of the preceding claims, wherein the first electrically conductive connection (17) connects the read-out contact (57) to the second electrically conductive connection (19) in an electrically conductive manner.

9. X-ray detector (1) according to one of claims 6 to 8, wherein the first electrically conductive connection (17) comprises a via connection between the read-out contact (57) and the intermediate contact (27), the intermediate contact (27), and a multilayer via connection between the intermediate contact (27) and the second electrically conductive connection (19).

10. X-ray detector (1) according to one of the preceding claims, wherein an active layer (5) of the integrated circuit is protected against direct incidence of light from the top side by the first light protection (7), the second light protection (9) or a third light protection (11) .

11. X-ray detector (1) according to claim 10, wherein the second light protection (9, 10) or the third light protection (10, 11) is a metal layer (10).

12. X-ray detector (1) according to claim 10 or 11, wherein the third light protection (11) has a gap.

13. X-ray detector (1) according to one of claims 10 to 12, wherein the third light protection (10, 11) is a metal layer (10) below the second light protection (9, 10).

14. X-ray detector (1) according to one of the preceding claims, wherein the second electrically conductive connection (19) is designed as a metal layer (10).

15. X-ray detector (1) according to one of the preceding claims, wherein a fourth light protection (13) is provided at the top side of the substrate (23).

16. X-ray detector (1) according to one of the preceding claims, wherein a second insulation layer (26) is provided at the top side of the substrate (23) and outside the surface of the read-out contact (57), and the fourth light protection (13) is covered at least partly by the second insulation layer (26).

17. X-ray detector (1) according to one of the preceding claims, wherein the first light protection (7), the first insulation layer (25), the second light protection (9), and an active layer (5) comprising the pre-amplifier (3) are sequentially disposed in a stacked arrangement.

18. X-ray detector (1) according to one of the preceding claims, wherein an upper metal layer (10), a middle metal layer (10) and a lower metal layer (10) are disposed in a stacked arrangement between the first insulation layer (25) and the active layers (5).

19. Medical apparatus having an X-ray detector (1) according to one of the preceding claims.

20. Medical apparatus according to claim 19, wherein the medical apparatus is a computed tomograph (31).

## Revendications

1. Détecteur (1) à rayons X comportant un substrat (23) ayant une liaison conductrice de l'électricité entre un contact (57) de lecture, qui est relié d'une manière conductrice de l'électricité à un capteur (53) par un joint brasé dans la partie de la face supérieure du substrat (23) et une entrée d'un préamplificateur (3) dans une couche (5) active d'un circuit intégré, dans lequel
a. une première liaison (17) conductrice de l'électricité est ménagée entre le contact (57) de lecture et une deuxième liaison (19) conductrice de l'électricité,
b. une surface d'une première protection (7) vis-à-vis de la lumière sur la face supérieure du substrat (23) est plus grande qu'une surface d'une partie (15) transparente à la lumière délimitée latéralement par une deuxième protection (9) vis-à-vis de la lumière, du substrat (23), de sorte que la surface de la première protection (7) vis-à-vis de la lumière recouvre la surface de la partie (15) transparente à la lumière dans une première projection suivant la normale à la surface et la partie (15) transparente à la lumière se trouve dans la partie offusquée par la première protection (7) vis-à-vis de la lumière,
c. la première protection (7) vis-à-vis de la lumière se trouve au-dessus du contact (57) de lecture sur la surface du substrat (23) ou la première protection (7) vis-à-vis de la lumière forme une unité avec le contact (57) de lecture,
d. la deuxième liaison (19) conductrice de l'électricité est ménagée à l'intérieur d'une deuxième projection de la surface de la partie (15) transparente à la lumière suivant la normale à la surface et en dessous de la deuxième protection (9) vis-à-vis de la lumière et
e. une troisième liaison (21) conductrice de l'électricité est ménagée en dessous de la deuxième protection (9) vis-à-vis de la lumière entre la deuxième liaison (19) conductrice de l'électricité et le préamplicateur,
de manière à protéger l'entrée du préamplicateur (3) de l'incidence directe de la lumière.

2. Détecteur (1) à rayons X suivant la revendication 1, dans lequel le contact (57) de lecture est la première protection (7) vis-à-vis de la lumière.

3. Détecteur (1) à rayons X suivant l'une des revendications précédentes, dans lequel la première liaison (17) conductrice de l'électricité ou la troisième liaison (21) conductrice de l'électricité est une liaison via.

4. Détecteur (1) à rayons X suivant la revendication 3, dans lequel la première liaison (17) conductrice de l'électricité ou la troisième liaison (21) conductrice de l'électricité est constituée, au moins en partie, sous la forme d'une liaison via à plusieurs couches.

5. Détecteur (1) à rayons X suivant l'une des revendications précédentes, dans lequel une première couche (25) isolante est mise entre la face supérieure du substrat (23) et la deuxième protection (9) vis-à-vis de la lumière/

6. Détecteur (1) à rayons X suivant l'une des revendications précédentes, dans lequel la première liaison (17) conductrice de l'électricité a un contact (27) intermédiaire, la deuxième protection (9) vis-à-vis de la lumière a plusieurs couches (10) métalliques et le contact (27) intermédiaire est mis dans une couche (10) métallique supérieure.

7. Détecteur (1) à rayons X suivant la revendication 6, dans lequel le contact (27) intermédiaire a, dans une projection suivant la normale à la surface, une surface plus petite que le contact (57) de lecture.

8. Détecteur (1) à rayons X suivant l'une des revendications précédentes, dans lequel la première liaison (17) conductrice de l'électricité relie, d'une manière conductrice de l'électricité, le contact (57) de lecture et la deuxième liaison (19) conductrice de l'électricité.

9. Détecteur (1) à rayons X suivant l'une des revendications 6 à 8, dans lequel la première liaison (17) conductrice de l'électricité a une liaison via entre le contact (57) de lecture et le contact (27) intermédiaire, le deuxième contact (27) intermédiaire est une liaison via à plusieurs couches entre le contact (27) intermédiaire et la deuxième liaison (19) conductrice de l'électricité.

10. Détecteur (1) à rayons X suivant l'une des revendications précédentes, dans lequel une couche (5) active du circuit intégré est protégée d'une incidence directe de la lumière par le côté supérieur par la première protection (7) vis-à-vis de la lumière, par la deuxième protection (9) vis-à-vis de la lumière ou par une troisième protection (11) vis-à-vis de la lumière.

11. Détecteur (1) à rayons X suivant la revendication 10, dans lequel la deuxième protection (9, 10) vis-à-vis de la lumière ou la troisième protection (10, 11) vis-à-vis de la lumière est une couche (10) métallique.

12. Détecteur (1) à rayons X suivant la revendication 10 ou 11, dans lequel la troisième protection (11) vis-à-vis de la lumière a un évidement.

13. Détecteur (1) à rayons X suivant l'une des revendications 10 à 12, dans lequel la troisième protection (10, 11) vis-à-vis de la lumière est une couche (10) métallique en dessous de la deuxième protection (9, 10) vis-à-vis de la lumière.

14. Détecteur (1) à rayons X suivant l'une des revendications précédentes, dans lequel la deuxième liaison (19) conductrice de l'électricité est constituée sous la forme d'une couche (10) métallique.

15. Détecteur (1) à rayons X suivant l'une des revendications précédentes, dans lequel une quatrième protection (13) vis-à-vis de la lumière est mise sur la face supérieure du substrat (23).

16. Détecteur (1) à rayons X suivant l'une des revendications précédentes, dans lequel une deuxième couche (26) isolante est mise sur la face supérieure du substrat (23) à l'extérieur de la surface du contact (57) de lecture et la quatrième protection (13) vis-à-vis de la lumière est recouverte, au moins en partie, de la deuxième couche (26) isolante.

17. Détecteur (1) à rayons X suivant l'une des revendications précédentes, dans lequel la première protection (7) vis-à-vis de la lumière, la première couche (25) isolante, la deuxième protection (9) vis-à-vis de la lumière et une couche (5) active ayant le préamplificateur (3) se succèdent suivant un empilement.

18. Détecteur (1) à rayons X suivant l'une des revendications précédentes, dans lequel sont disposées, entre la première couche (25) isolante et les couches (5) actives, une couche (10) métallique supérieure, une couche (10) métallique intermédiaire et une couche (10) métallique inférieure suivant un empilement.

19. Appareil médical comportant un détecteur (1) à rayons X suivant l'une des revendications précédentes.

20. Appareil médical suivant la revendication 19, dans lequel l'appareil médical est un tomographe (31) à ordinateur.
